# EUROPEAN PATENT SPECIFICATION

(11) **EP 3 325 625 B1**
(45) Date of publication and mention of the grant of the patent: **04.12.2019**
(21) Application number: 16757079.5
(22) Date of filing: 19.07.2016
(51) Int. Cl.: C12N 15/113, C12N 5/10, A01K 67/027

(54) **PROCESS FOR INDUCING RESISTANCE TO DIPHTHERIA TOXIN IN HUMAN CELLS, PRODUCTS AND USES THEREOF**
VERFAHREN ZUR INDUZIERUNG VON RESISTENZ GEGEN DIPHTHERIETOXIN IN MENSCHLICHEN ZELLEN, PRODUKTE UND VERWENDUNGEN DAVON
PROCÉDÉ PERMETTANT D'INDUIRE UNE RÉSISTANCE À LA TOXINE DIPHTÉRIQUE DANS DES CELLULES HUMAINES, PRODUITS ET UTILISATIONS DE CES DERNIERS

(30) Priority: 21.07.2015 IT UB20152371
(43) Date of publication of application: 30.05.2018
(73) Proprietor: Università Degli Studi Di Torino, 10122 Torino (IT)
(72) Inventor: MEDICO, Enzo, 10146 Torino (IT); PICCO, Gabriele, 10071 Borgaro Torinese (TO) (IT)
(74) Representative: Freyria Fava, Cristina
(86) International application number: PCT/IB2016/054293
(87) International publication number: WO 2017/013585

(56) References cited:
- WO-A1-2007/143858
- VINCENT ROY ET AL: "A Dominant-Negative Approach That Prevents Diphthamide Formation Confers Resistance to Pseudomonas Exotoxin A and Diphtheria Toxin", PLOS ONE, vol. 5, no. 12, 23 December 2010 (2010-12-23), page e15753, XP055245675, DOI: 10.1371/journal.pone.0015753 cited in the application
- GABRIELE PICCO ET AL: "A diphtheria toxin resistance marker for in vitro and in vivo selection of stably transduced human cells", SCIENTIFIC REPORTS, vol. 5, 30 September 2015 (2015-09-30), page 14721, XP055245105, DOI: 10.1038/srep14721

## Description

### FIELD OF THE INVENTION

The present invention concerns a process for inducing resistance to diphtheria toxin in human cells, products and uses thereof.

### BACKGROUND OF THE INVENTION

Stable transduction, i.e. permanent integration of exogenous genetic material in the cellular genome, is widely employed in basic and applied biomedical research.

In the case of human cells, this is typically achieved by transduction with an expression vector encoding resistance proteins for cytotoxic antibiotics like G418, puromycin, hygromycin and others. Pharmacologic selection then yields a cell population constitutively expressing the resistance marker, plus additional genetic elements of interest.

However, a major limitation of currently available selectable markers lies, in fact, in the impossibility to apply any selection process *in vivo:* the drugs used for selection are toxic for all eukaryotic cells and therefore would be lethal if administered to a mouse hosting human cells, e.g. a human tumor xenograft.

This restriction is not particularly relevant for cell lines, which can be transduced and selected *in vitro* and then implanted or injected into immunocompromised mice.

It is instead a crucial drawback for patient-derived cancer xenografts (PDXs), which are directly propagated from human tumour tissue into immunocompromised mice and for which no procedures for *in vivo* stable transduction have been found to work efficiently, with the exception of constructs that directly provide a selective advantage during outgrowth.

Such methodology is not suitable for basic tumor marking or for therapeutic proof-of-concept studies in which expression of the sequence of interest would instead be detrimental to cancer cells.

*Ex vivo* transduction strategies, by which cells are separated from explanted tissues, transduced and reimplanted, have also been described. However, the complexity and low efficiency of this approach pose severe limits to their practical application. Moreover, *in vitro* culture of PDX-derived cancer cells has been shown to deeply and irreversibly alter their gene expression profile, and therefore their functional state. WO 2007/143858 discloses a C-terminal deletion mutant of the DPH2 gene and its use for in vitro selection of transfected cells with DT.

Therefore, it is felt the need for the identification of new selectable markers usable in *in vivo* selection of human cells for marker studies or for therapeutic target validation.

### OBJECT AND SUMMARY OF THE INVENTION

The object of the present invention is to provide a new selectable marker conferring resistance to DT usable either in *in vitro* or in *in vivo* selection of human cells, wherein the *in vivo* selection is carried out in a rodent xenografted with human cells.

According to the invention, the above object is achieved thanks to the method specified in the ensuing claims, which are understood as forming an integral part of the present description.

The instant disclosure discloses a process for inducing resistance to diphtheria toxin in a human cell, wherein the process is carried out *in vitro* and/or *in vivo* in a rodent xenografted with a human cell, the process comprising the following steps:
a) providing at least one expression vector containing at least one nucleotide sequence targeting by RNA interference the DPH2 gene transcript of the human cell;
c) transducing the at least one vector into the human cell;
d) allowing transcription in the human cell, from the at least one expression vector, of the at least one nucleotide sequence targeting by RNA interference the DPH2 gene transcript;
e) obtaining silencing of the DPH2 gene in the human cell, whereby the human cell is resistant to diphtheria toxin.

The instant description discloses a transgenic diphtheria toxin resistant human cell comprising at least one expression vector containing at least one nucleotide sequence targeting by RNA interference the human DPH2 gene transcript, wherein transcription of the at least one expression vector silences the DPH2 gene in the human cell.

According to an embodiment, the instant description concerns use of an expression vector containing at least one nucleotide sequence targeting by RNA interference the DPH2 gene transcript of a human cell for inducing resistance to diphtheria toxin in a human cell, wherein the vector is used *in vivo* in a rodent xenografted with a human cell, wherein the transcript of the expression vector is permanently transduced in the human cell, whereby the permanently transduced human cell survives upon diphtheria toxin treatment. In said embodiment, the expression vector contains at least one additional nucleotide sequence, whereby the permanently transduced human cell surviving upon diphtheria toxin treatment contains the at least one additional nucleotide sequence.

### BRIEF DESCRIPTION OF THE DRAWINGS

The invention will now be described, by way of example only, with reference to the enclosed figures of drawing, wherein:

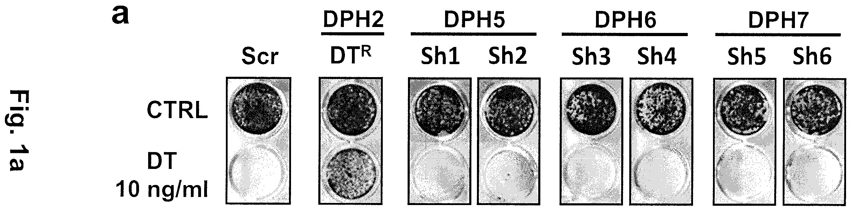

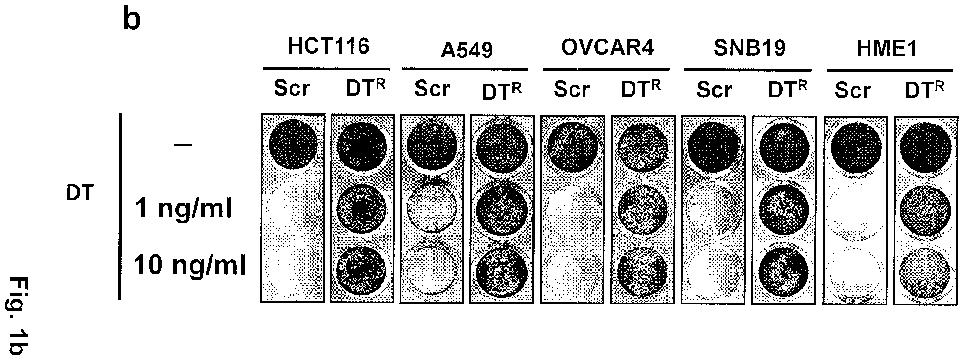

- **Figure 1****. *DPH2 silencing* renders human *cell lines resistant to DT.*** (**a**) Crystal violet staining of HCT116 cells transduced with Scramble or shRNAmirs targeting DPH2 (DT^{R}), DPH5 (sh1-2), DPH6 (sh3-4) and DPH7 (sh5-6), grown in the absence or presence of DT (10 ng/ml) for one week. The only construct conferring resistance to DT was DT^{R} (**b**) Crystal violet staining of human normal (HME1) and neoplastic cell lines (HCT116, colon; A549, lung; OVCAR4, ovary; SNB19, glioblastoma) transduced with Scramble or DT^{R} vector and grown in the absence or presence of DT (1 ng/ml or 10 ng/ml) for one week.

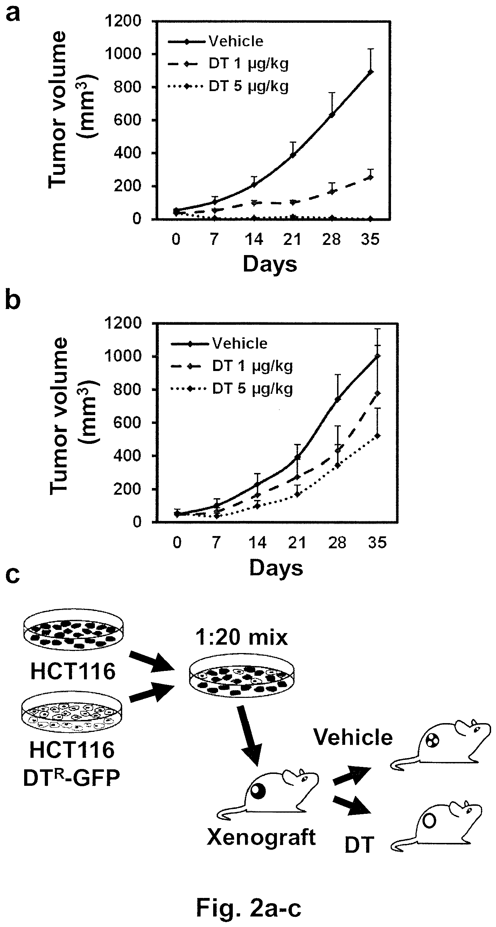

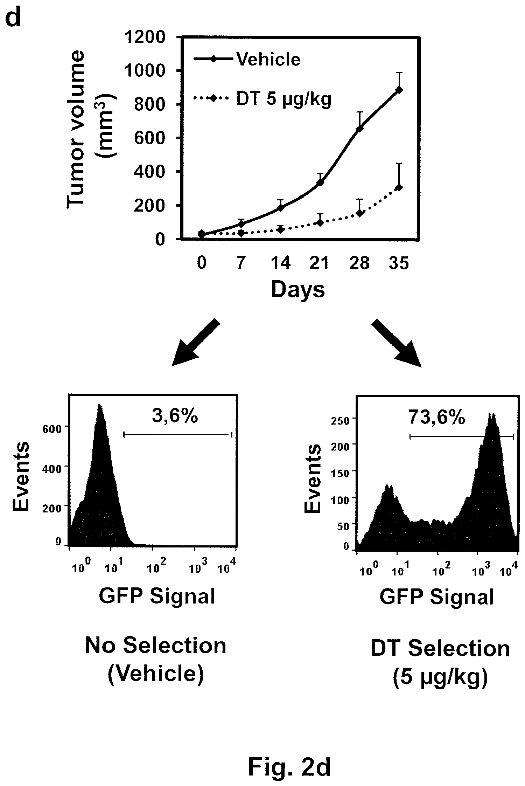

- **Figure 2****. DT^{R} transduced cells are resistant to DT in *vivo.*** (**a**,**b**) Tumor growth curves of xenografts from HCT116 cells transduced with either the Scramble vector (**a**) or the DT^{R} vector (**b**) in CD1-nude mice treated with DT (1 µg/kg or 5 µg/kg) or vehicle (n=5) from time 0 to day 21. (**c**) Schema of the *in vivo* selection experiment. CD1-nude mice xenografts obtained from a mixture of DT^{R}-transduced (GFP-positive) and parental (GFP-negative) HCT116 cells (1:20 ratio) were treated with DT (5 µg/kg) or vehicle for three weeks. It is expected that xenografts grown in the presence of DT are enriched in GFP-positive cells. (**d**) Tumor growth curves (n=4) in the course of the DT selection process, followed by flow-cytometry analysis of GFP levels in representative tumors explanted from unselected (left panel) or selected (right panel) cohorts, as indicated.

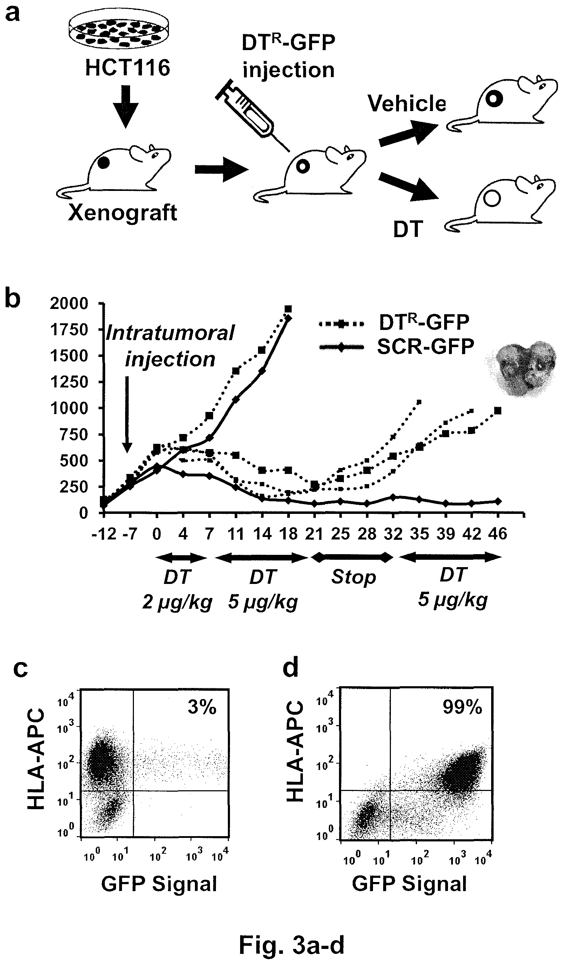

- **Figure 3****. In *vivo DT^{R} transduction and selection of transduced tumors.*** (**a**) Schema of the *in vivo* DT^{R} transduction and selection experiment. (**b**) Growth curves of HCT116 xenografts in CD1-nude mice transduced *in vivo* by intratumoral injection of scramble-GFP or DT^{R}-GFP concentrated lentiviral particles. A week after vector injection, DT was administered at increasing concentrations for three weeks, followed by 12 days of drug withdrawal and two additional weeks of treatment, as indicated. One control tumor for each vector was allowed to grow in the absence of DT as a control. (**c**,**d**) Flow cytometry analysis of the fraction of GFP+ cells in unselected (**c**) or DT-selected (**d**) tumors.

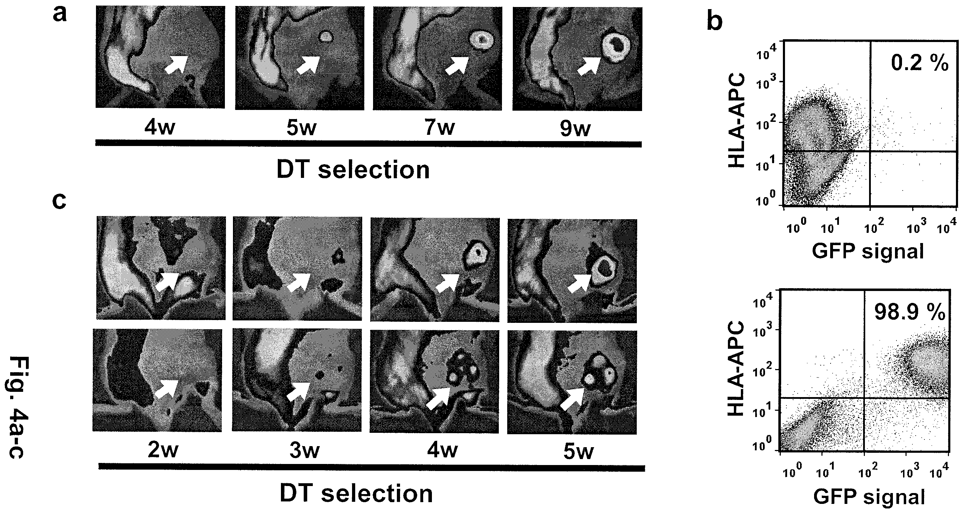

- **Figure 4****.** ***In vivo DT^{R}** t**ransduction and selection of CRC patient-derived xenografts.*** (**a**) Time-course live imaging during *in vivo* selection of a PDX grown in a NOD-SCID mouse and directly injected (white arrow) with the DT^{R}-GFP lentiviral vector. After about five weeks of selection, a GFP-positive area emerges in the tumor mass. (**b**) Flow cytometry analysis for GFP and human HLA-APC signals of unselected (top panel) or DT-selected PDXs (bottom panel), explanted at the end of the selection period. (**c**) Live imaging of an additional CRC PDX model *in vivo* injected with DT^{R}-GFP lentiviral particles in a single or multiple site (upper and lower panels, respectively) and selected with DT for five weeks. In this model, GFP-positive regions become already detectable after three weeks of selection.

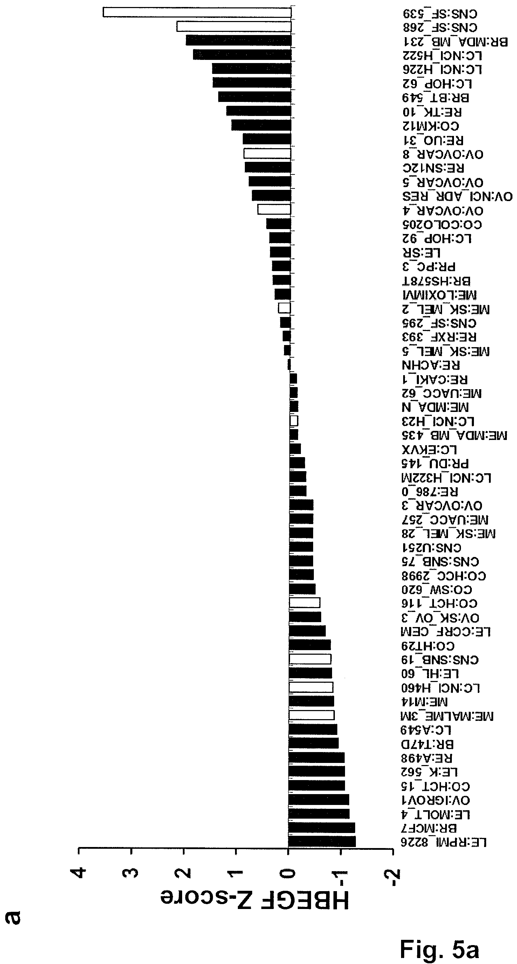

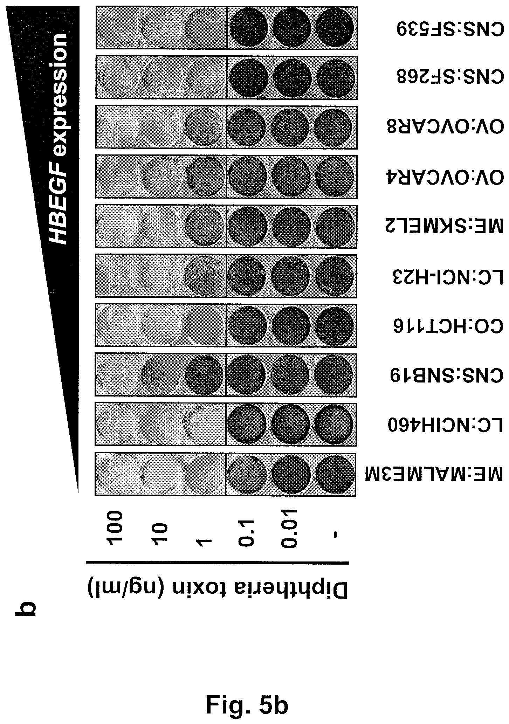

- ***Figure 5******. HBEGF expression does not affect sensitivity to DT in human cancer cell lines.*** (**a**) Histogram representing HBEGF mRNA expression (Z-score) in the NCI60 panel of human cancer cell lines (data from the cBbioPortal: http://www.cbioportal.org). White bars point out the ten cell lines from different tissues selected for DT treatment. **(**b) Crystal violet staining of selected cell lines, grown for one week in the presence of variable DT concentrations. Cells are ordered by increasing HBEGF expression, from left to right, as indicated by the black wedge on top.

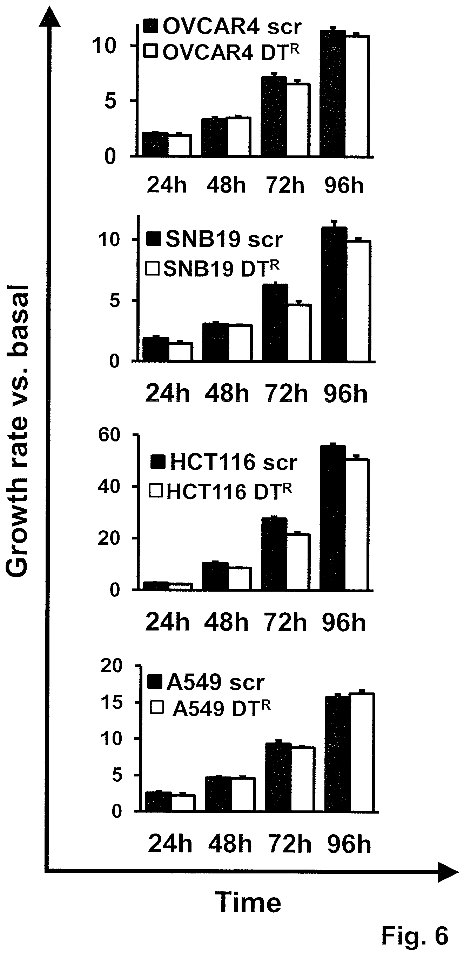

- ***Figure 6******. DPH2 silencing by DT^{R} does not affect basal growth* rate *of* cancer *cell lines.*** Histograms representing the growth of OVCAR4 (ovary), SNB19 (glioblastoma), HCT116 (colon) and A549 (lung) cancer cell lines transduced with DT^{R} or scramble construct. The growth rates on the y-axes were calculated by comparing ATP-based viability measurements at each time point vs. the measurements at plating.

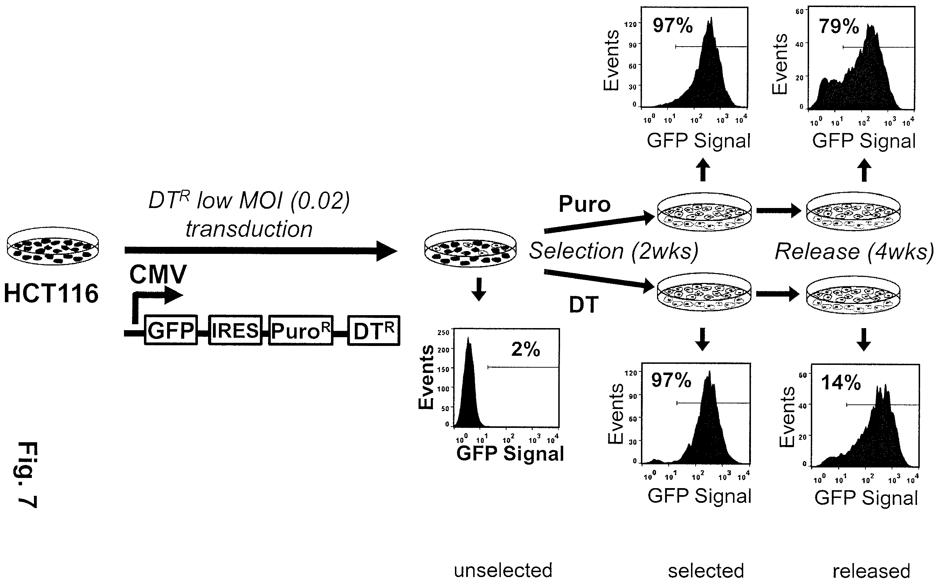

- ***Figure* 7****. DT^{R} *is* an efficient *selectable marker* in *vitro.*** HCT116 cells were infected with DT^{R} at low MOI (∼0.02), to ensure transduction of a low fraction of cells with a single copy of the vector. Three days after infection, only ∼2% of the cells expressed GFP. Cells were then incubated with puromycin (2 ng/ml) or DT (10 ng/ml) for two weeks. Subsequently, selection was released for four weeks to assess stability of the GFP+ fraction. Histograms represent the distribution of the GFP signal in unselected transduced cells, after puromycin/DT selection and after release from puromycin/DT selection, as indicated.

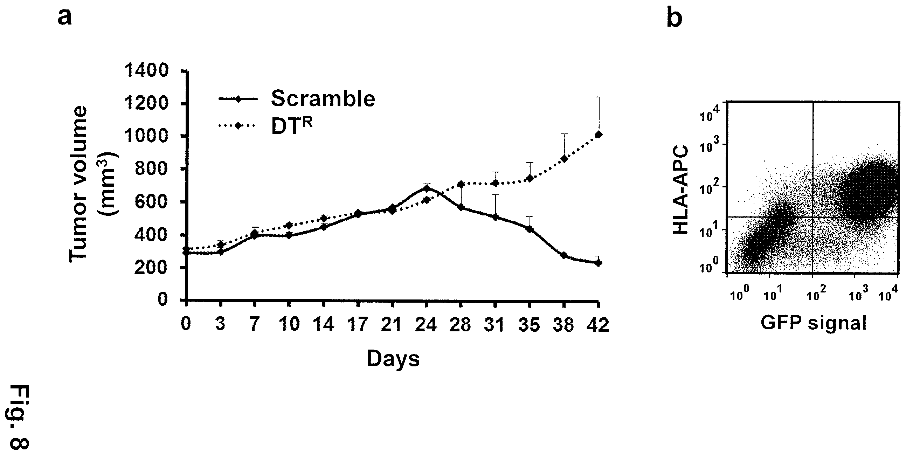

- ***Figure 8******. Stability of DT resistance and GFP expression after tumor propagation.*** (**a**) Scramble and DT^{R} transduced/selected HCT116 xenografts (n = 2 per group) were re-implanted in the right flank of CD1-nude mice. When tumors reached approximately a volume of 250 mm³, tumour growth was monitored for three weeks. At day 24, DT (5 µg/kg) was administered to mice. DT^{R} tumors (dotted line) continued growing in presence of DT, while scramble tumors (continuous line) rapidly reduced their volume. (**b**) Flow cytometry analysis of a DT^{R} xenograft explanted at day 24 (before the new DT selection) revealed a very high fraction of GFP+ cells.

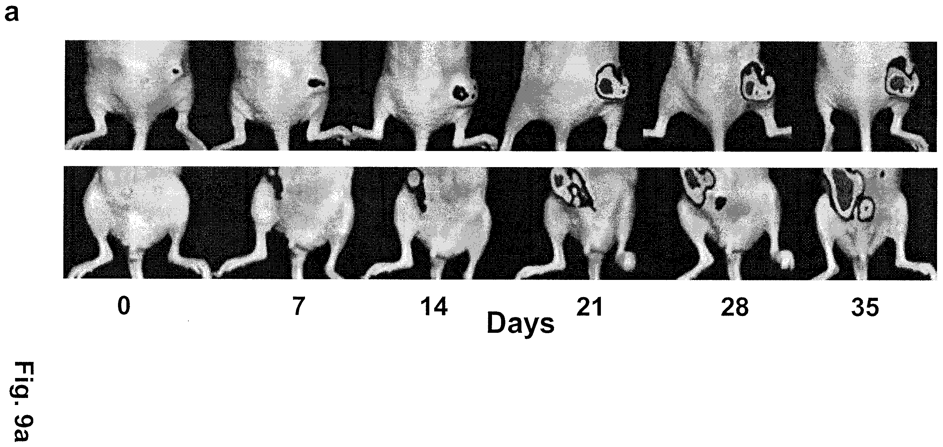

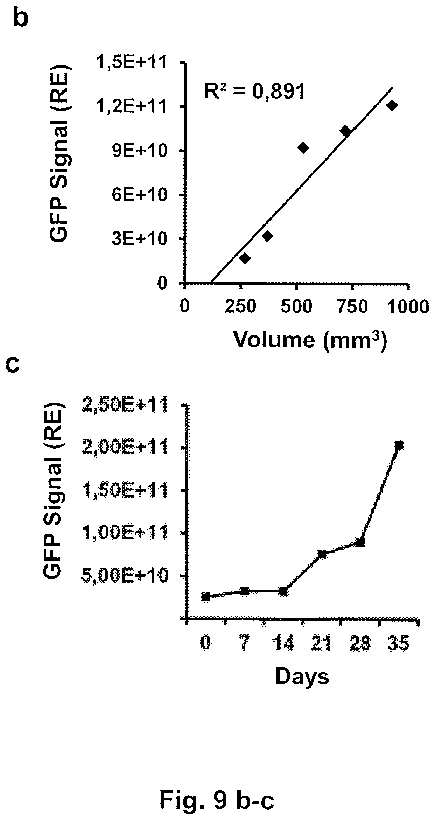

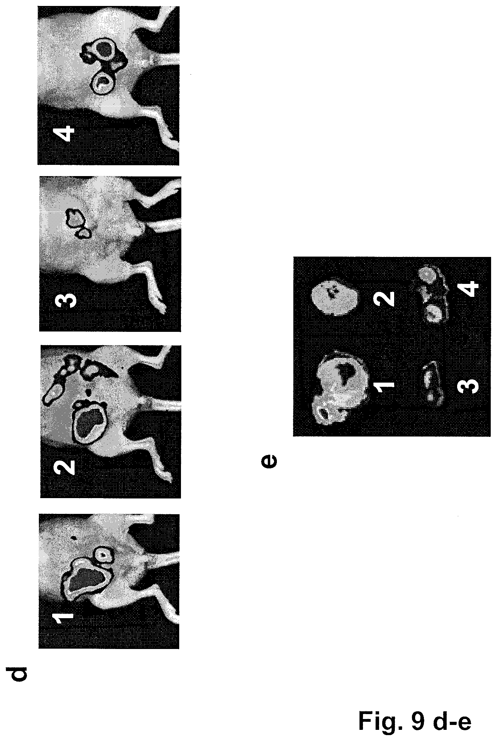

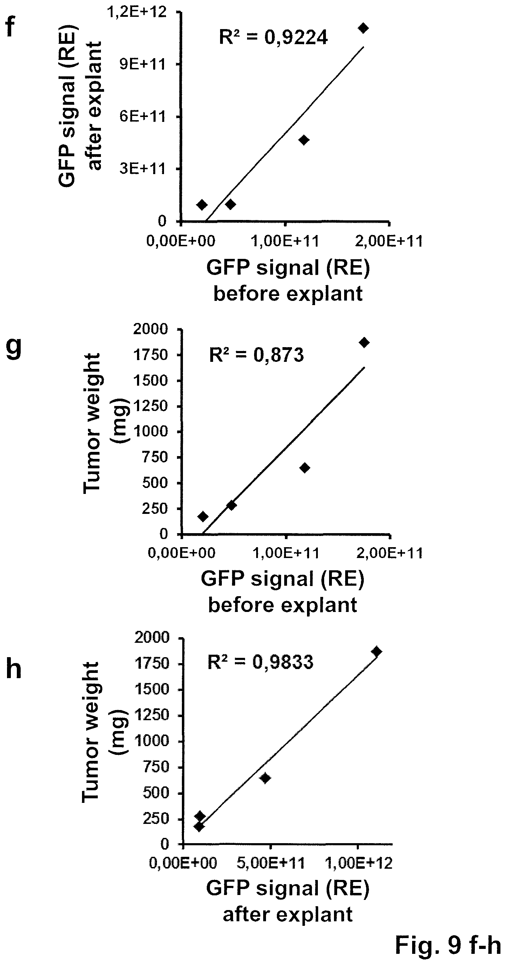

- ***Figure 9******.* Reliable *live imaging of DT^{R}***-***transduced HCT116 xenografts growing subcutaneously and intraperitoneally.*** (**a**) Live imaging of HCT116 xenografts transduced *in vivo* with DT^{R}-GFP, selected with DT and propagated subcutaneously (upper panels) or intraperitoneally (lower panels) in CD1-nude mice. (**b**) Scatter plot representing the correlation between caliper measurements (x-axis) and GFP fluorescence signal (y-axis; Radiant efficiency, RE) detected by live imaging in subcutaneous xenografts. (**c**) Time-course GFP signal measured by live imaging as a surrogate maker of intraperitoneal tumor growth. (**d**) Live imaging of four DT^{R} intraperitoneal xenografts. (**e**) GFP imaging of the tumours after explant. (**f**) Scatter plot comparing GFP signal before explant with GFP signal after explant or with tumor weight after explant. (**g**) Scatter plot comparing GFP signal before explant with tumor weight after explant. (**h**) Scatter plot comparing GFP signal after explant with tumor weight after explant.

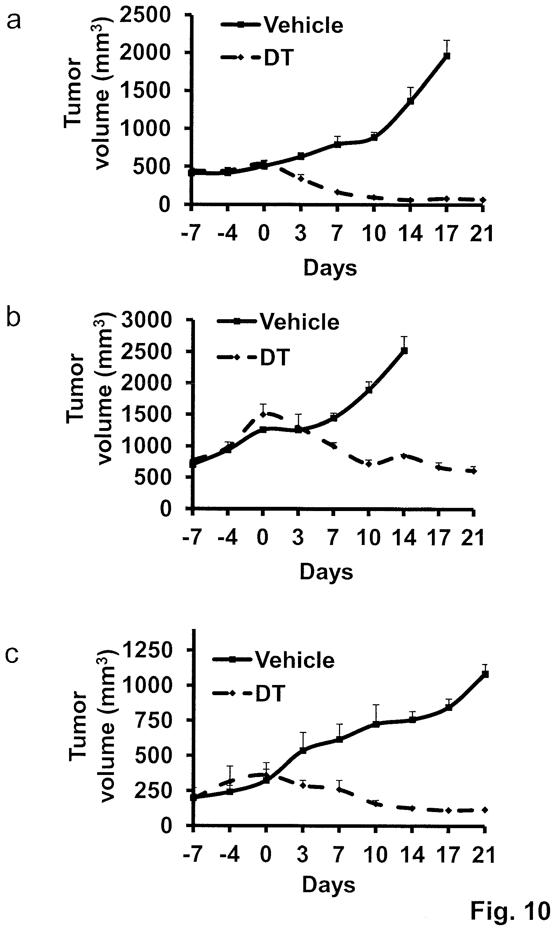

- ***Figure 10******. Colorectal* cancer *PDXs* are** ***sensitive** to DT.* PDXs from three metastatic CRC specimens, with different KRAS/BRAF genetic background were grown in NOD/SCID mice. The three graphs represent tumor growth inhibition of xenografts derived from a KRAS mutated (G13D) PDX (**a**), a BRAF mutated (V600E) PDX (**b**) or a KRAS/BRAF WT PDX (**c**), after administration of DT (10 µg/kg) for three weeks.

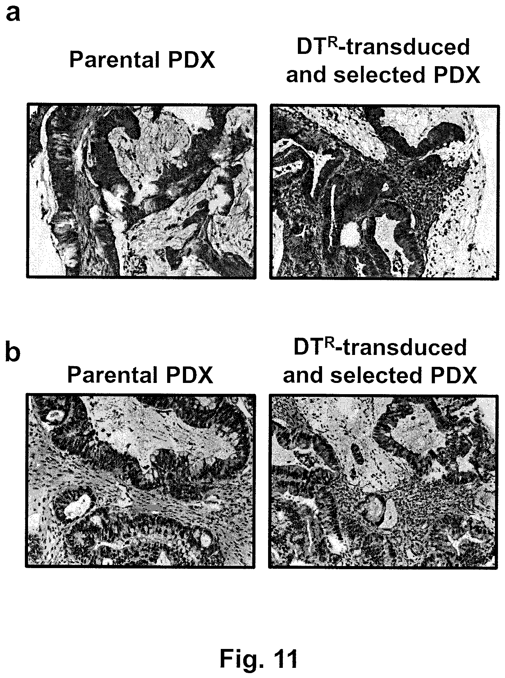

- ***Figure 11*****. *DT^{R}-transduced PDXs retain histopathologic and functional characteristics of the original sample.*** (**a**) Haematoxylin and eosin staining of parental and DT^{R}-transduced PDX. (**b**) Ki67 expression in parental and DT^{R}-transduced PDX.

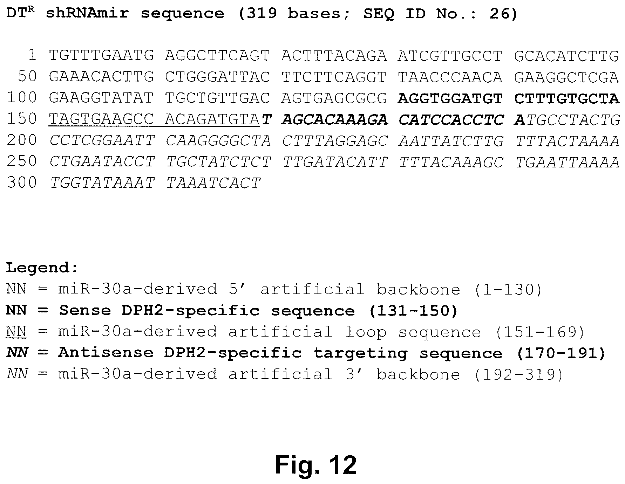

- ***Figure 12******. Sequence of the DT^{R} cassette.*** The DPH2-specific sense sequence (bold) and antisense targeting sequence (bold italic) are incorporated into an miR-30a-derived backbone for efficient transcription and processing.

### DETAILED DESCRIPTION OF THE INVENTION

The invention will now be described in detail, by way of non limiting example, with reference to a shRNAmir sequence able to silence DPH2 gene in human cells in order to obtain human cells which are resistant to diphtheria toxin.

It is clear that the scope of this description is in no way limited to the specifically disclosed shRNAmir sequence, being possible making use of nucleotide sequences targeting by RNA interference the DPH2 gene transcript of human cells incorporated in double stranded RNA, short hairpin RNA or microRNA.

In the following description, numerous specific details are given to provide a thorough understanding of embodiments. The embodiments can be practiced without one or more of the specific details, or with other methods, components, materials, etc. In other instances, well-known structures, materials, or operations are not shown or described in detail to avoid obscuring aspects of the embodiments.

Reference throughout this specification to "one embodiment" or "an embodiment" means that a particular feature, structure, or characteristic described in connection with the embodiment is included in at least one embodiment. Thus, the appearances of the phrases "in one embodiment" or "in an embodiment" in various places throughout this specification are not necessarily all referring to the same embodiment. Furthermore, the particular features, structures, or characteristics may be combined in any suitable manner in one or more embodiments.

The headings provided herein are for convenience only and do not interpret the scope or meaning of the embodiments.

The present description discloses a process for inducing resistance to diphtheria toxin in human cells, the process being usable either in *in vitro* or in *in vivo* selection of human cells, wherein the *in vivo* selection is carried out in a rodent xenografted with human cells.

The present description discloses a process for inducing resistance to diphtheria toxin in a human cell, wherein the process is carried out *in vitro* and/or *in vivo* in a rodent, preferably a mouse, xenografted with a human cell, the process comprising the following steps:
a) providing at least one expression vector containing at least one nucleotide sequence targeting by RNA interference the DPH2 gene transcript of the human cell;
c) transducing the at least one vector into the human cell;
d) allowing transcription in the human cell, from the at least one expression vector, of the at least one nucleotide sequence targeting by RNA interference the DPH2 gene transcript;
e) obtaining silencing of the DPH2 gene in the human cell, whereby the human cell is resistant to diphtheria toxin.

The transcript of the at least one expression vector is permanently transduced into the human cell.

The at least one nucleotide sequence targeting by RNA interference the DPH2 gene transcript is incorporated into: a double stranded RNA, a short hairpin RNA (shRNA) or a microRNA (shRNAmir).

The at least one nucleotide sequence targeting by RNA interference the DPH2 gene transcript comprises an antisense strand comprising at least 19 continuous bases of mRNA complementary to the DPH2 gene sequence set forth in SEQ ID No.: 1 and a matching sense strand.

The at least one nucleotide sequence targeting by RNA interference the DPH2 gene transcript comprises an antisense strand of 19-25 continuous bases, preferably 19-22 continuous bases, of mRNA complementary to the DPH2 gene sequence set forth in SEQ ID No.: 1 and a matching sense strand.

The at least one nucleotide sequence targeting by RNA interference the DPH2 gene transcript is selected from i) a double strand RNA composed of an antisense strand comprising a base sequence set forth in SEQ ID No.: 2, and a matching sense strand, the matching sense strand preferably comprising a base sequence set forth in SEQ ID No.: 3, which optionally has an overhang at the terminal of the antisense strand and/or sense strand, and ii) a double strand RNA composed of an antisense strand comprising a base sequence wherein one to several bases have been added to and/or deleted from the 5' terminal and/or 3' terminal of the base sequence described in SEQ ID No.: 2 and a matching sense strand, the matching sense strand preferably comprising a base sequence set forth in SEQ ID No.: 3, which optionally has an overhang at the terminal of the antisense strand and/or sense strand.

The at least one nucleotide sequence targeting by RNA interference the DPH2 gene transcript is operably linked to at least one regulatory sequence active in a human cell.

The regulatory sequence comprises a promoter sequence and/or a terminator sequence active in a human cell, being the promoter sequence a constitutive or foreign promoter sequence.

The present description discloses an isolated transgenic diphtheria toxin resistant human cell comprising at least one expression vector containing at least one nucleotide sequence targeting by RNA interference the human DPH2 gene transcript, wherein transcription of the at least one expression vector silences the DPH2 gene in the isolated human cell.

The at least one expression vector further comprises at least one additional nucleotide sequence.

According to an embodiment, the instant description concerns use of an expression vector containing at least one nucleotide sequence targeting by RNA interference the DPH2 gene transcript of a human cell for inducing resistance to diphtheria toxin in a human cell, wherein the vector is used *in vivo* in a rodent, preferably a mouse, xenografted with a human cell, wherein the transcript of the expression vector is permanently transduced in the human cell, whereby the permanently transduced human cell survives upon diphtheria toxin treatment, wherein the expression vector contains at least one additional nucleotide sequence, whereby the permanently transduced human cell surviving upon diphtheria toxin treatment contains the at least one additional nucleotide sequence.

In an embodiment, the expression vector is transduced into a human cell and subsequently the transduced human cell is xenografted into a rodent, preferably a mouse, for *in vivo* studies of the human cell. Preferably the human cell is a patient derived tumor cell. The at least one additional nucleotide sequence is selected from: i) nucleotide sequences encoding a fluorescent or bioluminescent protein, ii) nucleotide sequences encoding an enzymatic protein marker, iii) nucleotide sequences promoting loss-of-function of an endogenous gene of the permanently transduced human cell, and iv) nucleotide sequences promoting gain-of-function of an endogenous or exogenous gene in the permanently transduced human cell.

In an embodiment, when the at least one additional nucleotide sequence encodes a fluorescent or bioluminescent protein such a nucleotide sequence allows for imaging the permanently transduced human cell and/or monitoring response of the permanently transduced human cell to biological or chemical compounds, or physical treatments.

In a different embodiment, when the at least one additional nucleotide sequence promotes loss-of-function of an endogenous gene of the permanently transduced human cell, or promotes gain-of-function of an endogenous or exogenous gene in the permanently transduced human cell, such a nucleotide sequence allows for defining the biological function of the endogenous and/or exogenous gene in the permanently transduced human cell.

According to a further embodiment, the instant description provides for use of an expression vector containing at least one nucleotide sequence targeting by RNA interference the DPH2 gene transcript of a human cell for inducing resistance to diphtheria toxin in a human cell, wherein the transcript of the expression vector is permanently transduced in the human cell, whereby the permanently transduced human cell survives upon diphtheria toxin treatment for tumor marking, clinical evaluation of anti-tumor therapeutic sequences and/or identification of new oncogenic markers.

In an embodiment, the instant disclosure concerns a method for *in vivo* selection of human cells (preferably patient derived tumor cells) xenografted in a rodent (preferably a mouse), wherein the human cells are rendered resistant to DT by using an expression vector containing at least one nucleotide sequence targeting by RNA interference the DPH2 gene transcript of the human cells, wherein the transcript of the expression vector is permanently transduced in the human cells, whereby the permanently transduced human cells survive upon diphtheria toxin treatment.

In a preferred embodiment, the method for *in vivo* selection of human cells xenografted in a rodent provides for using an expression vector further containing at least one additional nucleotide sequence selected from: i) nucleotide sequences encoding a fluorescent or bioluminescent protein, ii) nucleotide sequences encoding an enzymatic protein marker, iii) nucleotide sequences promoting loss-of-function of an endogenous gene of the permanently transduced human cell, and iv) nucleotide sequences promoting gain-of-function of an endogenous or exogenous gene in the permanently transduced human cell.

In a further preferred embodiment, the method for *in vivo* selection of human cells xenografted in a rodent provides for using an expression vector further containing at least one additional nucleotide sequence selected from: i) nucleotide sequences encoding a fluorescent or bioluminescent protein, and ii) nucleotide sequences encoding an enzymatic protein marker, wherein the at least one additional nucleotide sequence allows for detecting the human cells in the rodent.

In an embodiment, the human cells are transduced with the expression vector either before (i.e. *in vitro*) or after (i.e. *in vivo*) being xenografted in the rodent.

Within the present description, the expression "matching sense strand" means reverse complementary sequence, accepting a limited number of mismatches as commonly found in double stranded RNAs, short hairpin RNAs or microRNAs.

The present inventors reasoned that, in principle, injection of a viral expression vector into the patient-derived cancer xenograft (PDX) mass followed by treatment of the mouse with a drug exerting species-specific killing activity only on human non-transduced cells would enable selection of stably transduced PDXs, for marker studies or for proof-of-concept therapeutic target validation.

The present inventors considered that diphtheria toxin (DT) has the required pharmacological properties: it invariably kills human cells using the ubiquitously expressed transmembrane heparin-binding EGF-like growth factor (HBEGF) as a receptor, but has no effect on mouse tissues, because the murine Hbegf does not bind DT. Moreover, DT has been successfully employed for cell lineage ablation in animal models. A well-documented strategy to induce DT resistance in human cells is the blockade of Diphthamide Biosynthesis Protein 2 (DPH2), either by expression of a dominant-negative protein (1) or by gene inactivation via insertional mutagenesis (2). However, both approaches to DPH2 blockade are not optimal resistance markers: (1) expression of a dominant-negative protein would require insertion in the expression vector of a promoter or internal ribosome entry site, typically longer than 500 nucleotides, plus the dominant-negative DPH2 full coding sequence (398 aminoacids, i.e. 1194 nucleotides), accounting in total for more than 1700 nucleotides, which would limit the size of additional elements to be inserted in the vector; (2) insertional mutagenesis is a random, non-directed event, and therefore cannot be programmed to occur in all transduced cells.

DPH2 catalyzes a key step in diphthamide biosynthesis, a histidine modification process known to occur only on His715 of Eukaryotic Elongation Factor 2 (EEF2). After HBEGF-mediated DT internalization, DT inhibits EEF2 by catalyzing the transfer of NAD+ to diphthamide. In the absence of active DPH2, His715 is not converted to diphthamide and the cell is insensitive to DT. Additional genes, including DPH5, DPH6, and DPH7, have been shown to encode proteins essential for diphthamide formation and DT-mediated toxicity in human cells. Notably, cells lacking diphthamide have no distinct phenotypes except their resistance to DT. Therefore, the interruption of diphthamide biosynthesis represents an attractive strategy to render human cells resistant to DT without major biological consequences.

To develop a selectable marker conferring resistance to DT, the present inventors considered an RNA interference approach, using - as an embodiment - a short hairpin sequences inserted into a primary microRNA transcript backbone (shRNAmirs). This design adds a Drosha processing site to the hairpin construct that has been shown to greatly increase knockdown efficiency (3). One or two different shRNAmir sequences were tested for each of the key diphthamide biosynthesis genes.

The present inventors demonstrated that an artificial shRNAmir sequence targeting the DPH2 transcript invariably confers resistance to DT in human cells, and that this sequence can be effectively exploited to select permanently transduced human cells both *in vitro* and *in vivo.*

According to a preferred embodiment, the shRNAmir sequence adopted by the present inventors and identified in the following as DT^{R} sequence (SEQ ID No.: 26 also shown in Figure 12) has two advantageous features: (i) it is only 319 nucleotides long, considering the entire mir-30 backbone of the DT^{R} shRNAmir; (ii) it can be directly inserted between the promoter and the coding sequence of interest, or after its stop codon, before the polyadenylation site. In this way, the nascent transcript is processed to yield both the mRNA of interest and the DT^{R} marker, without the need for additional promoters or internal ribosome entry sites. The instant results collected using DT^{R} sequence show that this is the case: with DT^{R}, preferably inserted downstream from GFP, all DT-selected cells displayed strong GFP fluorescence.

At odds with the above described features, currently used selectable markers encode for proteins capable of rendering transduced cells resistant to a toxic compound. The coding sequence for such markers is several hundred nucleotides long, and requires a dedicated promoter or an internal ribosome entry site for efficient translation, which substantially increases vector size. This in turn reduces the size of additional genetic elements that can be efficiently cloned and transduced.

A straightforward way to render human cells resistant to DT would be to abrogate expression of HBEGF, encoding the transmembrane DT receptor. However, due to its mitogenic activity and to its release as a soluble form after membrane shedding, its silencing is likely to induce phenotypic changes in both transduced and surrounding cells. Similarly, other enzymes required for diphthamide formation, like DPH1 and DPH3, are not suitable candidates because they have been involved in the regulation of cell growth and development.

Among the remaining candidate targets, DPH2 was not the only one tested in our screening for DT resistance markers: shRNAmirs were tested against each of the DPH5, DPH6 and DPH7 genes. None of them was capable of inducing detectable DT resistance. Indeed, the extent of target mRNA downregulation was quite limited for most of them.

*In vivo* stable transduction of human cells with vectors containing nucleotide sequence(s) targeting by RNA interference the DPH2 gene transcript, and further containing other nucleotide sequences, like for example fluorescent or bioluminescent proteins, is expected to significantly improve the spectrum and performance of tumor-tracking applications.

This is particularly true for intraperitoneal and other orthotopic models, where caliper measurements are not suitable, and fluorescence or bioluminescence imaging represent a cost effective, simple approach.

The process to induce resistance to diphtheria toxin in human cells disclosed herein enables in fact robust expression of other co-selected coding sequences, as confirmed by the high levels of GFP signal detected by flow cytometry and fluorescence microscopy in selected cells and xenografts, wherein the growth of DT^{R}-GFP transduced tumors was effectively monitored by *in vivo* imaging, even when the xenografts were intraperitoneal.

Additional possible applications of the process to induce resistance to diphtheria toxin in human cells disclosed herein include transduction of PDXs also with other biologically or therapeutically relevant sequences, like e.g. cDNAs for proteins modulating drug response, or short RNA hairpins interfering with gene expression of undruggable or poorly actionable oncogenic drivers.

In light of the rapidly increasing availability and use of PDX models for preclinical experimentation, the system to induce resistance to diphtheria toxin in human cells presented here offers an unprecedented opportunity for direct genetic manipulation of human tumor xenografts.

Such transduced xenografts, after *in vivo* selection with DT, may be useful for several applications, depending on the additional genetic elements introduced in the vector containing nucleotide sequence(s) targeting by RNA interference the DPH2 gene transcript in human cells, as here exemplified:
i. if the additional genetic element is a marker (fluorescent, bioluminescent, enzymatic protein or other): follow by vital imaging local and disseminated tumor growth under various conditions, to monitor the activity of pro- or anti-oncogenic molecules and treatments to which the transduced human cells are exposed;
ii. if the additional genetic element promotes the loss-of-function of an endogenous gene of interest in the transduced cells (by RNA interference, recombination, dominant-negative action, or other): follow local and disseminated tumor growth under various conditions, to define the biological function of the targeted gene of interest (e.g. pro- or anti-oncogenic function), and optionally its interactions with pro- or anti-oncogenic molecules and treatments to which the transduced human cells are exposed;
iii. if the additional genetic element promotes the gain-of-function of a gene of interest in the transduced cells (by cDNA overexpression, recombination, mutation or other): follow local and disseminated tumor growth under various conditions, to define the biological function of the targeted gene of interest (e.g. pro- or anti-oncogenic function), and optionally its interactions with pro- or anti-oncogenic molecules and treatments to which the transduced human cells are exposed.

To the above identified aims, development of efficient multi-module expression vectors will be greatly facilitated by the small size and pri-miRNA nature of DT^{R}.

### Results

### DPH2 silencing renders human cells resistant to diphtheria toxin in vitro.

To validate DT as a selective agent, the present inventors verified its activity on a panel of cell lines of different tissue origin, expressing variable levels of the DT receptor, HBEGF (4). DT activity was indeed always high and independent of HBEGF expression levels (Figure 5). To assess if silencing of the genes involved in diphthamide biosynthesis confers resistance to DT, HCT116 colorectal cancer (CRC) cells, expressing intermediate levels of HBEGF, were chosen as a model and transduced with 18 different shRNAmir constructs, targeting the DPH2, DPH5, DPH6 and DPH7 transcripts.

Table 1 provides details of the gene-specific portions of the shRNAmir constructs employed in the screening. For each shRNAmir, columns report the targeted gene, the lentiviral vector system employed and the mature siRNA antisense sequence, which is the active one guiding the RISC complex to specific mRNA degradation. The DT^{R} construct is highlighted in bold.

**Table 1**

| **Clone ID** | **Gene** | **ID** | **Vector** | **Active mature sequence** | **Seq Id No. :** |
|---|---|---|---|---|---|
| **V3LHS_382955** | **DPH2** | **DT^{R}** | **pGIPZ** | **A: AGCACAAAGACATCCACCT** | **2** |
| | | | | **S: AGGTGGATGTCTTTGTGCT** | **3** |
| V3THS_318423 | DPH5 | Sh1 | pTRIPZ | A: TGAACAATCTCCAGAAGCT | 4 |
| | | | | S: AGCTTCTGGAGATTGTTCA | 5 |
| V3THS_318422 | DPH5 | Sh2 | pTRIPZ | A: TGATTTTGAACAATCTCCA | 6 |
| | | | | S: TGGAGATTGTTCAAAATCA | 7 |
| V3LHS_319554 | DPH6 | Sh3 | pGIPZ | A: TGTCTTGTATCCAAGCTCC | 8 |
| | | | | S: GGAGCTTGGATACAAGACA | 9 |
| V3LHS_319557 | DPH6 | Sh4 | pGIPZ | A: AGATTTGCTAAAGCAACGA | 10 |
| | | | | S: TCGTTGCTTTAGCAAATCT | 11 |
| V2THS_212240 | DPH7 | Sh5 | pTRIPZ | A: TTTAAGAGAAGTCAACAGC | 12 |
| | | | | S: GCTGTTGACTTCTCTTAAA | 13 |
| V3THS_301465 | DPH7 | Sh6 | pTRIPZ | A: AATTGAAAGCAGCAATCCA | 14 |
| | | | | S: TGGATTGCTGCTTTCAATT | 15 |

Transduced cells were then tested for their response to DT (Figure 1a) and for downregulation of the target transcript (Table 2). The tested shRNAmir targeting DPH2 was found to induce both robust downregulation of DPH2 mRNA levels and strong resistance to DT. This shRNAmir sequence is hereafter referred to as DT^{R} ("diphtheria toxin resistance"). Efficient downregulation of DPH2 and induction of resistance to DT was confirmed in three additional human cancer cell lines derived from different tissues and in a non-transformed human breast epithelial cell line (Figure 1b, Table 2). Notably, cells expressing the DT^{R} marker were not impaired in their growth rate (Figure 6). To assess if DT^{R} can be efficiently employed as a selectable marker *in vitro,* the present inventors exploited the expression cassette for GFP and puromycin resistance included in the lentiviral DT^{R} vector. HCT116 were transduced with DT^{R} at low MOI to obtain around 2% of GFP-positive (GFP+) cells, and then selected in the presence of either DT (10 ng/ml) or puromycin (2 ng/ml) for two weeks. Both selections were found to strongly increase the GFP+ fraction (>95%; Figure 7). To verify the stability of the resistant phenotype, both selected populations were grown in the absence of DT or puromycin for one month, and found to retain a very high fraction of GFP+ cells (∼80% and ∼90% for puromycin and DT-selected cells, respectively; Figure 7). Persistence of the high GFP+ fraction was also observed after multiple freeze-thaw cycles.

### Human cells transduced with DT^{R} become resistant to diphtheria toxin in vivo.

To assess if DT^{R}-transduced cells maintain DT resistance also in the context of a living organism, HCT116 cells transduced with DT^{R} or with a control vector were grown as subcutaneous xenografts in nude mice. When xenografts reached ∼50 mm³, mice were treated with DT or with vehicle for three weeks. As shown in Figure 2a, treatment of control HCT116 xenografts with 1 µg/kg DT strongly reduced their growth rate, and 5 µg/kg DT induced complete tumor regression, which persisted also after DT suspension.

Conversely, DT^{R} transduced cells resisted to both doses of DT and continued growing in the presence of DT and after its withdrawal (Figure 2b). Notably, in the absence of DT, DT^{R}-transduced HCT116 xenografts displayed a growth rate similar to that of control xenografts (Figure 2a and 2b, "Vehicle" lines), confirming that DT^{R} expression has no major effects on cancer cell growth. No adverse effects were observed in mice treated with DT.

To verify the possibility of using DT^{R} as a marker for *in vivo* selection of transduced cells, the present inventors designed the experiment illustrated in Figure 2c.

Briefly, DT^{R}-GFP-transduced and control HCT116 cells were mixed in a 1:20 ratio, to obtain a heterogeneous population in which the DT^{R}-expressing, GFP+ fraction was around 5%. The mixed cell population was then implanted and grown in nude mice xenografts in the absence or presence of DT for three weeks, followed by two additional weeks without treatment. As shown in Figure 2d, treatment with 5 µg/kg DT did not induce tumor regression but only a reduced growth rate.

After two weeks from the end of the treatment, flow cytometry on the explanted tumors revealed a striking enrichment of GFP+ cells in the DT-treated arm (Figure 2d).

Altogether, these results show that DPH2 silencing by DT^{R} confers resistance to DT also *in vivo* and can be exploited for *in vivo* selection of transduced cells.

### In vivo transduction and selection for DT^{R} expression in xenografts from human cell lines and tumors.

Generation of genetically modified xenografts is easily accomplishable with most neoplastic cell lines by *in vitro* transduction and selection, followed by implant in mice.

In the case of PDXs however, such procedure is typically not applicable or poorly efficient.

The present inventors therefore sought to verify if direct intratumoral injection of the DT^{R} vector in xenografts from human cell lines and patient-derived tumors, followed by DT treatment of mice, could lead to the development of xenografts significantly enriched in transduced cells.

To this aim, HCT116 xenografts in nude mice were directly transduced by intratumoral injection of concentrated lentiviral particles of DT^{R}-GFP or control(scramble)-GFP vector.

After one week, cell suspensions were obtained from a first set of transduced tumors for flow cytometry analysis, which detected a fraction of GFP+ cells around 1% for both vector types (average of 5 measurements = 0.97% +/- 1.70 %). In a parallel set of xenografts, one week after transduction, mice were treated with DT for three weeks, followed by two weeks of suspension, after which DT was maintained until tumor explant (Figure 3a).

While all tumors displayed marked shrinkage after three weeks of DT treatment, only DT^{R}-transduced tumors resumed growth after the initial shrinkage. The regrown tumors featured a multinodular mass, suggestive of parallel growth of multiple resistant subclones from the areas of vector injection (Figure 3b, photo insert). DT^{R}-GFP transduced and selected xenografts revealed a striking enrichment in human GFP+ cells with respect to transduced tumors grown in the absence of selection (99% vs 3%; Figure 3c,d).

Intriguingly, the high fraction of transduced cells and the DT resistance were stably maintained over multiple passages in mice, also in the absence of DT selective pressure (Figure 8). Moreover, growth of DT^{R}-GFP transduced tumors implanted subcutaneously or in the peritoneum cavity could be longitudinally followed by measuring *in vivo* GFP fluorescence signal as a surrogate value of the tumor mass volume (Figure 9).

As a proof of concept of the efficiency of the DT^{R} vector system in an additional preclinical platform, the present inventors reproduced the same experimental procedure employing human CRC PDXs instead of cell line xenografts.

A preliminary test confirmed that also these PDX tumors were sensitive to DT, independently of their genetic makeup (Figure 10). One CRC PDX model was transduced *in vivo* by a single intratumoral injection of DT^{R}-GFP lentiviral particles. Also in this case transduction efficiency was estimated as above to be around 1%.

The DT selection process was then monitored by *in vivo* tracking of the GFP signal in the transduced tumors. To reduce the background due to the white fur, the skin surrounding the xenograft was shaved. After about five weeks of selection, the GFP signal became detectable in the tumor mass, and kept increasing in the following weeks (Figure 4a). As confirmed by flow cytometry, almost all human cancer cells were positive for GFP (Figure 4b). Also in this case lentiviral transduction of PDX tumors with DT^{R}-GFP did not alter tumor morphology or proliferative index (Ki67 expression; Figure 11).

Efficiency of the DT^{R} selection procedure was confirmed in a second PDX model, transduced with single or multiple injections of DT^{R}-GFP lentiviral particles. During DT selection, single or multiple GFP+ areas became detectable, reflecting growth of GFP+ cells in proximity of the injection sites (Figure 4c).

Also in this case, the selection process therefore led to the formation of a growing GFP+ tumor.

### Materials and Methods

### Cell culture and reagents

The following cell lines were purchased from American Type Culture Collection (ATCC): HCT-116 (ATCC_CCL-247), A549 (ATCC_CCL-185), SNB-19 (ATCC_CRL2219) and HME1 (ATCC_CRL4010). OVCAR-4 cells were purchased from the Cell Line Repository of the National Cancer Institute's Division of Cancer Treatment and Diagnosis (NCI DCTD). Cell lines were maintained in culture following supplier guidelines, and routinely screened for absence of Mycoplasma contamination using the Venor® GeM Classic kit (Minerva biolabs). The identity of each cell line was checked by Cell ID™ System and by Gene Print® 10 System (Promega), throught Short Tandem Repeats (STR) at 10 different loci (D5S818, D13S317, D7S820, D16S539, D21S11, vWA, TH01, TPOX, CSF1PO and amelogenin). Cells were ordinarily supplemented with FBS at different concentrations, 2mM L-glutamine, antibiotics (100U/mL penicillin and 100 mg/mL streptomycin) and grown in a 37°C and 5% CO₂ air incubator. In particular, HCT-116 and A549 cell line were maintained in RPMI-1640 medium, supplemented with 10% FBS. SNB-19 and OVCAR4 cells were maintained in DMEM, supplemented with 10% FBS. HME1 cells were cultured in DMEM/F-12 supplemented with 20 ng/mL EGF, 10 µg/mL insulin, and 100 µg/mL hydrocortisone and 10% FBS. Proliferation assays were performed seeding 5-15 x 10³ cells in 24-well plates or 5 x 10⁴ cells in 6 well plate. After 24 hours, medium was replaced adding DT (D0564, Sigma) reconstituted to 0.5 mL with sterile distilled water. After one week of treatments, cells were fixed with a solution of 3% Paraformaldehyde plus 1% glucose and then stained with 0.05% Crystal Violet in Distilled Water.

### Gene sequences

Human DHP2 gene transcript has the nucleotide sequence set forth in SEQ ID NO.: 1, also disclosed in Genbank under accession number NM_001384.4; human DHP5 gene transcript has the nucleotide sequence disclosed in Genbank under accession number NM_001077394.1; human DHP6 gene transcript has the nucleotide sequence disclosed in Genbank under accession number NM_080650.3; human DHP7 gene transcript has the nucleotide sequence disclosed in Genbank under accession number NM_138778.3.

### RNA interference

For stable silencing, pGIPZ shRNAmir vectors targeting DPH2 (V3LHS_382955) and DPH6 (V3LHS_319554 and V3LHS_319557), together with control pGIPZ scrambled-shRNAmir vectors, were purchased from Dharmacon (http://dharmacon.gelifesciences.com). For inducible silencing, pTRIPZ shRNAmir vectors targeting DPH5 (V3THS_318423 and V3THS_318422) and DPH6 (V2THS_212240 and V3THS_301465), plus control pTRIPZ scrambled-shRNAmir vector were purchased from Dharmacon (http://dharmacon.gelifesciences.com) .

Table 2 provides detailed information about extent of target transcripts downregulation achieved in cell lines after transduction with lentiviral shRNAmir constructs, analyzed by quantitative real-time PCR. For each cell line, it is reported the target gene, the clone ID of the lentiviral vector employed, the fraction of residual mRNA and the standard deviation values. Data obtained with the DT^{R} construct are highlighted in bold. Indeed, only few shRNAmirs were found to decrease the target transcripts of more than 50%: one for DPH2 and DPH5, one for DPH6 and none for DPH7. The bottom four lines report the silencing activity of DT^{R} in additional cell lines.

**Table 2**

| **Cell line** | **Gene Symbol** | **Clone ID** | **% of residual transcript** | **Standard Deviation** |
|---|---|---|---|---|
| **HCT116** | **DPH2** | **DT^{R}** | **14%** | **2%** |
| HCT116 | DPH5 | Sh1 | 47% | 2% |
| HCT116 | DPH5 | Sh2 | 87% | 4% |
| HCT116 | DPH6 | Sh3 | 40% | 5% |
| HCT116 | DPH6 | Sh4 | 27% | 4% |
| HCT116 | DPH7 | Sh5 | 110% | 14% |
| HCT116 | DPH7 | Sh6 | 110% | 6% |
| **A549** | **DPH2** | **DT^{R}** | **10%** | **1%** |
| **OVCAR4** | **DPH2** | **DT^{R}** | **7%** | **1%** |
| **SNB19** | **DPH2** | **DT^{R}** | **5%** | **0%** |
| **HME1** | **DPH2** | **DT^{R}** | **6%** | **1%** |

The Open Biosystems protocols were followed for bacterial culture growth and for recombination checks. pGIPZ and pTRIPZ plasmids were purified with Miniprep or Maxiprep kits (Qiagen) and DNA concentration was measured by Nanodrop 1000. All lentiviral constructs were transfected into HEK293T cells plated in a 6-well plate at a density of 5 x 10⁴ cells per well one day prior to transfection, using Lipofectamine 2000 (Invitrogene). Supernatants were harvested after 48 hours, filtered through a 0.45-µm filter, and used to infect cells in the presence of 4 µg/mL of polybrene. Cells were selected in 2 µg/mL puromycin or directly in diphtheria toxin at the indicated doses. For cells transduced with inducible pTRIPZ vector, shRNAmir expression was induced adding doxycycline (1 µg/mL) in to the growth medium and RFP fluorescence was assessed by FACS analysis before performing growth assay and Q-RT-PCR.

For *in vivo* injection, supernatants of 7 15-cm plates of transfected HEK293 cells were concentrated by ultracentrifugation. Concentration of the viral p24 antigen was determined by HIV-1 p24 core profile ELISA (PerkinElmer Life Sciences).

### Q-RT-PCR

Total RNA was extracted using the miRNeasy Mini Kit (Qiagen), according to the manufacturer's protocol.

The quantification and quality analysis of RNA was performed by Thermo Scientific Nanodrop 1000 and Bioanalyzer 2100 (Agilent). DNA was transcribed using iScript RT Super Mix (BioRad) following the manufacturer's instructions.

Q-RT-PCR was performed in triplicate on ABI PRISM 7900HT thermal cycler (Life Technologies) with SYBR green dye. The sequences of the primers (Sigma Aldrich) used for gene expression analyses of DPH2, DPH5, DPH6 and DPH7 genes are reported in Table 3.

**Table 3**

| **Gene** | **Sequence FW 5'** | **Sequence RV 5'** |
|---|---|---|
| DPH2 | CGTGCTTCGTCAACGTTCTG SED ID No.:16 | TGGGTTCTGGGCCTCAAA SED ID No.:17 |
| DPH5 | CCAGAAAGCTTCTTTGACAAAGTG SED ID No.:18 | GATTTTCCAAAGACTGCTCCTTTACT SED ID No.:19 |
| DPH6 | GATCCTGATAAGCATCTTGGGAAA SED ID No.:20 | CTCCACCTTCTCCACAAACATG SED ID No.:21 |
| DPH7 | CCTCTTGGGCTTGGCAGAT SED ID No.:22 | CAGGGCAAGGCTGGACAAT SED ID No.:23 |
| PGK | AGCTGCTGGGTCTGTCATCCT SED ID No.:24 | TGGCTCGGCTTTAACCTTGT SED ID No.:25 |

### Flow cytometry

GFP expression analysis of *in vitro* cultured cells was performed by flow cytometry: cells were trypsinized, diluted in a 1% paraformalhdeide-2% FBS solution, stained with DAPI (D9542, Sigma) and analyzed with FACS flow cytometer (CyAn., DAKO).

Explanted tumors were dissected by bistoury and resuspended in collagenase (C5138, Sigma), Trypsin and Medium 199 (Sigma), for 30 minutes at 37°C. After inhibition of tripsine and centrifugation, pellets were washed with HBSS 5% FBS and resuspended in red blood cell lysis buffer (NH₄Cl (155 nM), KHCO₃ (10 mM), EDTA (0,1 mM)) for 3 minutes. After incubation with DNAse (5ug/ml) for 5 minutes at 37°C, cells were counted and 10 x 10⁶ cells were stained with human HLA-ABC antibody (555555, BD Pharmingen) for 45 min in ice. Subsequently, for flow cytometry analyses, cells were filtered with 40 µm filters and stained with DAPI.

### Animal models and live imaging

Female, 6-8 week (20-25 grams) non-obese diabetic/severe combined immunodeficient (NOD/SCID) and CD-1 nude mice were purchased from Charles River Laboratories (Calco, Italy) and maintained in hyperventilated cages.

HCT116 xenografts were established by subcutaneous inoculation of 2 x 10⁶ cells into the right posterior flank of 5-6 week-old mice. Tumor size was evaluated by caliper measurements and the approximate volume of the mass was calculated using the formula (d/2)2 x D/2, where d is the minor tumor axis and D is the major tumor axis.

HCT116 secondary grafts, employed for *in vivo* transduction, were obtained starting from material explanted from HCT116 primary xenograft: tumors were cut into 25 to 30 mm³ pieces and re-implanted in the flank of the mice.

Each intratumoral injection was performed with approximately 1 mg of lentiviral particles diluted in physiological saline solution. Tumor cells were injected subcutaneously into immunodeficient mice as described above (2x10⁶ cells/mouse).

After 2 weeks, mice bearing tumors of approximately 100 mm³ were selected and were randomly divided into 3 groups of 6 mice each.

*In vivo* transduction experiments were conducted injecting vectors expressing the shRNAmir sequences and GFP reporter directly into the tumor mass. Single or multiple intratumoral injection of 1 mg of lentiviral particles were performed by a 0,5 ml syringe. The same procedure was employed to transduce cell line-derived xenografts and PDXs. In the latter case, tumor samples were procured for PDX engraftment from patients that provided informed consent, and the study was conducted under the approval of the institutional review board.

For selection of cell xenografts, DT was administered three times a week at the indicated dosages and schedules. For PDX selection, DT was administered three times a week (5 µg/kg) until tumor explant.

*In vivo* quantitative and qualitative analysis of GFP expression was performed by using the IVISR Lumina II imaging and the Living Image software version 3.0 (Caliper Life Science). The GFP filter sets in the system include emission filter (515-575 nm) and excitation filter (445-490 nm). Images of the mice were generated by setting, respectively, 1,5E⁹ and 1E¹⁰ as minimum and maximum values in the color scale. The ROI measurements were individually marked as regions of interest (ROI) in the Living Image software, setting auto ROI detection with 20 as auto detection threshold.

### Immunohistochemistry and confocal microscopy

To detect GFP expression, explanted tumors were fixed by immersion in formalin for 24 hours at 4°C. Subsequently, the specimens were treated with 30% sucrose solution to protect them from cryopreservation, then embedded in Optimal Cutting Temperature (OCT) compound (Bio-Optica) and stored at -80°C until sectioning. Subsequently, 12 µm thick sections were cut with a cryomicrotome (Leica CM 3050 S) and collected in Superfrost Plus Slides (Thermo Scientific). Before staining, microscope slides were treated with PBS to remove OCT and stained with DAPI (Sigma). The fluorescent light emitted by Turbo-GFP and DAPI was evaluated on a Leica TCS SP2 AOBS confocal microscope. Images were generated with the LAS AF Leica Application Suite software (Leica) at 20X of magnification. For IHC, formalin-fixed, paraffin-embedded tissues explanted from PDX were partially sectioned (10 µm thick) using a microtome. 4-µm paraffin tissue sections were dried in a 37°C oven overnight. Slides were deparaffinized in xylene and rehydrated through graded alcohol to water. Endogenous peroxidase was blocked in 3% hydrogen peroxide for 30 minutes. Microwave antigen retrieval was carried out using a microwave oven (750 W for 10 minutes) in 10 mmol/L citrate buffer, pH 6.0. Slides were incubated with monoclonal mouse anti-human Ki67 (clone MIB-1; DAKO) overnight at 4°C inside a moist chamber. Immunohistochemically stained slides for Ki67, were scanned with a x400 objective.

### Study Approval

All animal procedures and care administered were approved by the Ethical Committee of the University of Turin and the Italian Ministry of Health.

### REFERENCES

1. Roy V, Ghani K, and Caruso M. A dominant-negative approach that prevents diphthamide formation confers resistance to Pseudomonas exotoxin A and diphtheria toxin. PLoS One. 2010;5(12):e15753.
2. Carette JE, Guimaraes CP, Varadarajan M, Park AS, Wuethrich I, Godarova A, Kotecki M, Cochran BH, Spooner E, Ploegh HL, et al. Haploid genetic screens in human cells identify host factors used by pathogens. Science. 2009;326(5957):1231-5.
3. Boden D, Pusch O, Silbermann R, Lee F, Tucker L, and Ramratnam B. Enhanced gene silencing of HIV-1 specific siRNA using microRNA designed hairpins. Nucleic Acids Res. 2004;32(3):1154-8.
4. Reinhold WC, Sunshine M, Liu H, Varma S, Kohn KW, Morris J, Doroshow J, and Pommier Y. CellMiner: a web-based suite of genomic and pharmacologic tools to explore transcript and drug patterns in the NCI-60 cell line set. Cancer Res. 2012;72(14):3499-511.

## Claims

1. Use of an expression vector containing at least one nucleotide sequence targeting by RNA interference the DPH2 gene transcript of a human cell for inducing resistance to diphtheria toxin in a human cell, wherein the expression vector is used *in vivo* in a rodent xenografted with a human cell, wherein the transcript of the expression vector is permanently transduced in the human cell, whereby the permanently transduced human cell survives upon diphtheria toxin treatment, wherein the expression vector contains at least one additional nucleotide sequence, whereby the permanently transduced human cell surviving upon diphtheria toxin treatment contains the at least one additional nucleotide sequence, and wherein the at least one additional nucleotide sequence is selected from: i) nucleotide sequences encoding a fluorescent or bioluminescent protein, ii) nucleotide sequences encoding an enzymatic protein marker, iii) nucleotide sequences promoting loss-of-function of an endogenous gene of the permanently transduced human cell, and iv) nucleotide sequences promoting gain-of-function of an endogenous or exogenous gene in the permanently transduced human cell.

2. Use according to claim 1, wherein the at least one additional nucleotide sequence allows for imaging the permanently transduced human cell and/or monitoring response of the permanently transduced human cell to biological or chemical compounds, or physical treatments.

3. Use according to claim 1, wherein the at least one additional nucleotide sequence allows for defining the biological function of the endogenous and/or exogenous gene in the permanently transduced human cell.

4. Use according to any one of claims 1 to 3, for tumor marking, clinical evaluation of anti-tumor therapeutic sequences and/or identification of new oncogenic markers.

## Patentansprüche

1. Verwendung eines Expressionsvektors, der mindestens eine Nukleotidsequenz enthält, die durch RNA-Interferenz auf das DPH2-Gentranskript einer menschlichen Zelle abzielt, um eine Resistenz gegen Diphtherietoxin in einer menschlichen Zelle zu induzieren, wobei der Expressionsvektor *in vivo* in einem Nagetier verwendet wird, das mit einer menschlichen Zelle xenotransplantiert ist, wobei das Transkript des Expressionsvektors permanent in der menschlichen Zelle transduziert ist, wobei die permanent transduzierte menschliche Zelle nach Diphtherietoxinbehandlung überlebt, wobei der Expressionsvektor mindestens eine zusätzliche Nukleotidsequenz enthält, wobei die permanent transduzierte menschliche Zelle, die nach Diphtherietoxinbehandlung überlebt, die mindestens eine zusätzliche Nukleotidsequenz enthält, und wobei die mindestens eine zusätzliche Nukleotidsequenz ausgewählt ist aus: i) Nukleotidsequenzen, die ein fluoreszierendes oder biolumineszierendes Protein kodieren, ii) Nukleotidsequenzen, die einen enzymatischen Proteinmarker kodieren, iii) Nukleotidsequenzen, die den Funktionsverlust eines endogenes Gens der permanent transduzierten menschlichen Zelle fördern, und iv) Nukleotidsequenzen, die den Funktionsgewinn eines endogenen oder exogenen Gens in der permanent transduzierten menschlichen Zelle fördern.

2. Verwendung nach Anspruch 1, wobei die mindestens eine zusätzliche Nukleotidsequenz die Abbildung der permanent transduzierten menschlichen Zelle und/oder die Überwachung der Reaktion der permanent transduzierten menschlichen Zelle auf biologische oder chemische Verbindungen oder physikalische Behandlungen ermöglicht.

3. Verwendung nach Anspruch 1, wobei die mindestens eine zusätzliche Nukleotidsequenz die Definition der biologischen Funktion des endogenen und/oder exogenen Gens in der permanent transduzierten menschlichen Zelle ermöglicht.

4. Verwendung nach einem der Ansprüche 1 bis 3 zur Tumormarkierung, klinischen Bewertung von antitumoralen therapeutischen Sequenzen und/oder Identifizierung neuer onkogener Marker.

## Revendications

1. Utilisation d'un vecteur d'expression contenant au moins une séquence de nucléotides ciblant par interférence d'ARN le produit de transcription du gène DPH2 d'une cellule humaine pour induire une résistance à la toxine diphtérique dans une cellule humaine, dans laquelle le vecteur d'expression est utilisé *in vivo* dans un rongeur xénogreffé d'une cellule humaine, dans laquelle le produit de transcription du vecteur d'expression est transduit en permanence dans la cellule humaine, moyennant quoi la cellule humaine transduite de façon permanente survit suite à un traitement de la toxine diphtérique, dans laquelle le vecteur d'expression contient au moins une séquence de nucléotides additionnelle, moyennant quoi la cellule humaine transduite de façon permanente survivant suite à un traitement de la toxine diphtérique contient l'au moins une séquence de nucléotides additionnelle, et dans laquelle l'au moins une séquence de nucléotides additionnelle est choisie parmi : i) des séquences de nucléotides codant une protéine fluorescente ou bioluminescente, ii) des séquences de nucléotides codant un marqueur protéique enzymatique, iii) des séquences de nucléotides favorisant une perte de fonction d'un gène endogène de la cellule humaine transduite de façon permanente, et iv) des séquences de nucléotides favorisant un gain de fonction d'un gène endogène ou exogène dans la cellule humaine transduite de façon permanente.

2. Utilisation selon la revendication 1, dans laquelle l'au moins une séquence de nucléotides additionnelle permet l'imagerie de la cellule humaine transduite de façon permanente et/ou la surveillance de la réponse de la cellule humaine transduite de façon permanente à des composés biologiques ou chimiques, ou à des traitements physiques.

3. Utilisation selon la revendication 1, dans laquelle l'au moins une séquence de nucléotides additionnelle permet la définition de la fonction biologique du gène endogène et/ou exogène dans la cellule humaine transduite de façon permanente.

4. Utilisation selon l'une quelconque des revendications 1 à 3, pour le marquage d'une tumeur, l'évaluation clinique de séquences thérapeutiques antitumorales et/ou l'identification de nouveaux marqueurs oncogéniques.


### SEQUENCE LISTING

<110> Università degli Studi di Torino
<120> Process for inducing resistance to diphtheria toxin in human cells, products and uses thereof
<130> BWO18350-CF
<160> 26
<170> PatentIn version 3.5
<210> 1
   <211> 2513
   <212> DNA
   <213> homo sapiens
<400> 1
   cccggtagtc ccacgtgtag cggagaaaca gtagttagga tggctgaagg ggatactcac      60
   cggctgaagg ccgactgtga ttccccctac ccccacaagg cgattttgac cccctgaggg     120
   ctgctctaga ggactcaggc cccgaagctg tcccagggag gtccccgctg catcccacca     180
   cccaagctgt gcctcatgga gtcgatgttt agcagccctg ccgaggcggc gctgcagcga     240
   gagaccgggg tgccaggact gcttactcct cttccggacc tggacggagt gtacgagctg     300
   gagcgagtcg ctggatttgt ccgcgacctg gggtgtgaac gagttgcctt gcagttccct     360
   gaccagctat tgggagatgc tgtggctgtg gctgcacgac tggaggagac gacagggtca     420
   aagatgttca ttctgggtga cacagcctac ggcagctgct gcgtggatgt gctgggtgct     480
   gagcaagctg gagctcaggc tctcatacat tttggccctg cctgcttaag ccctccagcc     540
   cgcccactgc ccgttgcctt cgtgcttcgt caacgttctg tggccttgga gctctgtgtc     600
   aaggcctttg aggcccagaa cccagacccc aaagcgcctg tggtgctgct gagtgagccg     660
   gcctgtgccc atgccctgga ggctttggct actctcctgc gcccacggta cctggacctg     720
   ctagtctcca gcccagcttt tccccaacca gtgggttccc tgagtccaga gcctatgccc     780
   ctagagcgtt ttgggcgccg cttccccctt gccccaggga ggcgtctaga agagtatggt     840
   gccttctatg tagggggctc taaggccagc cctgacccag accttgaccc agacctgagt     900
   cggctgctct tggggtgggc accaggtcaa cccttctcct cctgctgtcc agatacaggg     960
   aagactcagg atgagggtgc ccgggctgga cggctaaggg cacgaagacg atatctggta    1020
   gagagggcca gagatgcccg cgtggtaggg ctgctggcag gcacactggg tgtagcccaa    1080
   caccgtgagg cactggccca cttgcggaac ctgactcagg ctgctggcaa gcgtagctat    1140
   gtgttggccc tggggcggcc cacccctgcc aagcttgcca acttccctga ggtggatgtc    1200
   tttgtgctat tagcctgtcc tctgggtgct ctagcccccc agctttctgg tagcttcttc    1260
   cagcctatac tggcaccatg tgagctggaa gctgcctgca accctgcctg gccacctcca    1320
   ggcctggctc cccacctcac acattatgcg gacttattgc ctggctctcc cttccacgtg 1380
   gctctcccac cacctgagtc agagctgtgg gaaaccccag acgtgtcact cattactgga 1440
   gatctccgac ccccacctgc ctggaagtca tcaaatgatc atggaagctt ggctctgacc 1500
   ccacggcccc agctggagct ggctgagagc agtcctgcag cctcattcct tagttcccgg 1560
   agctggcaag ggctggagcc ccgcctgggt cagacgccag tgacagaagc tgtgagtgga 1620
   agacgaggga ttgccatcgc ctatgaggat gagggaagcg gctgatacca tgtggggctg 1680
   gagacataga tggacttatg aatggctgct aggaccttta gtgctccctg caccaacctc 1740
   ccatccccct gccaagatcc ttgaaggacc ctggaaggag ggagagcagg cagcccttca 1800
   caggatagga tccgtctctg tcctgtcctg gcactggcac aagctcagca catgcccagt 1860
   aatgcgtgtt gtttggctga tggaataaag ggcttaggga cttccctgag gcctctggac 1920
   ccatctgtct tcctgagggc agcccaggac ctttggccaa tcccagttcc caggctgcag 1980
   ttgagggtct gtccttgtca aaaggcaggt gctagacagt ctagaccagg gtttctcaaa 2040
   ctcgtacttg acatttgggg ccagataatt ctttgttgtg gggctgtctg gtgtatggta 2100
   gggtgctcag cagcatccct ggcctctgcc cactagacat cagaagcact cccccagttg 2160
   tgacaaccaa aaatatctcc agaccttggc aaatgttatc tgtgggggaa aattgccctc 2220
   aattgagaac cactggtcta gctagacctg cactgtccag tacagtagcc actaaataca 2280
   tgtggctaaa cttaaattta agttaattaa gattaaaagc tcagtttctc agtcacatta 2340
   gtcattcaag tgttcagaca gccacatgag gggacagtgc agctacagga tatgccatca 2400
   tggcagaaag ttctattggt tggacagcgt tggtctatac tgactcttat ttctcaggga 2460
   gatcacagca acctaaataa accagatacc ttttctcaaa aaaaaaaaaa aaa 2513
<210> 2
   <211> 19
   <212> DNA
   <213> artificial
<220>
   <223> siRNA - DHP2 antisense strand
<400> 2
   agcacaaaga catccacct 19
<210> 3
   <211> 19
   <212> DNA
   <213> artificial
<220>
   <223> siRNA - DHP2 sense strand
<400> 3
   aggtggatgt ctttgtgct 19
<210> 4
   <211> 19
   <212> DNA
   <213> artificial
<220>
   <223> siRNA - DHP5 antisense
<400> 4
   tgaacaatct ccagaagct 19
<210> 5
   <211> 19
   <212> DNA
   <213> artificial
<220>
   <223> siRNA - DHP5 sense strand
<400> 5
   agcttctgga gattgttca 19
<210> 6
   <211> 19
   <212> DNA
   <213> artificial
<220>
   <223> siRNA - DHP5 antisense II
<400> 6
   tgattttgaa caatctcca 19
<210> 7
   <211> 19
   <212> DNA
   <213> artificial
<220>
   <223> siRNA - DHP5 sense II
<400> 7
   tggagattgt tcaaaatca 19
<210> 8
   <211> 19
   <212> DNA
   <213> artificial
<220>
   <223> siRNA- DHP6 antisense
<400> 8
   tgtcttgtat ccaagctcc 19
<210> 9
   <211> 19
   <212> DNA
   <213> artificial
<220>
   <223> siRNA - DHP6 sense
<400> 9
   ggagcttgga tacaagaca 19
<210> 10
   <211> 19
   <212> DNA
   <213> artificial
<220>
   <223> siRNA - DHP6 sense II
<400> 10
   agatttgcta aagcaacga 19
<210> 11
   <211> 19
   <212> DNA
   <213> artificial
<220>
   <223> siRNA - DHP6 sense II
<400> 11
   tcgttgcttt agcaaatct 19
<210> 12
   <211> 19
   <212> DNA
   <213> artificial
<220>
   <223> siRNA - DHP7 antisense
<400> 12
   tttaagagaa gtcaacagc 19
<210> 13
   <211> 19
   <212> DNA
   <213> artificial
<220>
   <223> siRNA - DHP7 sense
<400> 13
   gctgttgact tctcttaaa 19
<210> 14
   <211> 19
   <212> DNA
   <213> artificial
<220>
   <223> siRNA - DHP7 antisense II
<400> 14
   aattgaaagc agcaatcca 19
<210> 15
   <211> 19
   <212> DNA
   <213> artificial
<220>
   <223> siRNA - DHP7 sense II
<400> 15
   tggattgctg ctttcaatt 19
<210> 16
   <211> 20
   <212> DNA
   <213> artificial
<220>
   <223> DHP2 forward primer
<400> 16
   cgtgcttcgt caacgttctg 20
<210> 17
   <211> 18
   <212> DNA
   <213> artificial
<220>
   <223> DHP2 reverse primer
<400> 17
   tgggttctgg gcctcaaa 18
<210> 18
   <211> 24
   <212> DNA
   <213> artificial
<220>
   <223> DHP5 forward primer
<400> 18
   ccagaaagct tctttgacaa agtg 24
<210> 19
   <211> 26
   <212> DNA
   <213> artificial
<220>
   <223> DHP5 reverse primer
<400> 19
   gattttccaa agactgctcc tttact 26
<210> 20
   <211> 24
   <212> DNA
   <213> artificial
<220>
   <223> DHP6 forward primer
<400> 20
   gatcctgata agcatcttgg gaaa 24
<210> 21
   <211> 22
   <212> DNA
   <213> artificial
<220>
   <223> DHP6 reverse primer
<400> 21
   ctccaccttc tccacaaaca tg 22
<210> 22
   <211> 19
   <212> DNA
   <213> artificial
<220>
   <223> DHP7 forward primer
<400> 22
   cctcttgggc ttggcagat 19
<210> 23
   <211> 19
   <212> DNA
   <213> artificial
<220>
   <223> DHP7 reverse primer
<400> 23
   cagggcaagg ctggacaat 19
<210> 24
   <211> 21
   <212> DNA
   <213> artificial
<220>
   <223> PGK forward primer
<400> 24
   agctgctggg tctgtcatcc 21
<210> 25
   <211> 20
   <212> DNA
   <213> artificial
<220>
   <223> PGK reverse primer
<400> 25
   tggctcggct ttaaccttgt 20
<210> 26
   <211> 319
   <212> DNA
   <213> artificial
<220>
   <223> DTR sequence
<400> 26
   tgtttgaatg aggcttcagt actttacaga atcgttgcct gcacatcttg gaaacacttg 60
   ctgggattac ttcttcaggt taacccaaca gaaggctcga gaaggtatat tgctgttgac 120
   agtgagcgcg aggtggatgt ctttgtgcta tagtgaagcc acagatgtat agcacaaaga 180
   catccacctc atgcctactg cctcggaatt caaggggcta ctttaggagc aattatcttg 240
   tttactaaaa ctgaatacct tgctatctct ttgatacatt tttacaaagc tgaattaaaa 300
   tggtataaat taaatcact 319